(19) Europäisches Patentamt
European Patent Office
Office européen des brevets

(11) Numéro de publication: 0 056 766 B1

# (12) FASCICULE DE BREVET EUROPEEN

(45) Date de publication du fascicule du brevet: **16.05.84**

(51) Int. Cl.³: **C 07 D 215/42,** C 07 D 215/44, C 07 D 215/46 // A61K31/47, C07D215/22

(21) Numéro de dépôt: **82400080.6**

(22) Date de dépôt: **15.01.82**

## (54) Procédé de préparation d'amino-4 chloroquinoléines.

(30) Priorité: **16.01.81 FR 8100765**

(43) Date de publication de la demande: **28.07.82 Bulletin 82/30**

(45) Mention de la délivrance du brevet: **16.05.84 Bulletin 84/20**

(84) Etats contractants désignés: **AT BE CH DE FR GB IT LI LU NL SE**

(56) Documents cités: **US - A - 2 653 940**

(73) Titulaire: **RHONE-POULENC SANTE, Les Miroirs 18 Avenue d'Alsace, F-92400 Courbevoie Cedex (FR)**

(72) Inventeur: **Baudouin, Michel, 78, rue Parmentier, F-69190 St Fons (FR)**
Inventeur: **Michelet, Daniel, 24 Chemin de Montribloud, F-69160 Tassin (FR)**

(74) Mandataire: **Pilard, Jacques et al, RHONE-POULENC RECHERCHES Service Brevets Pharma 25, Quai Paul Doumer, F-92408 Courbevoie Cedex (FR)**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

EP 0 056 766 B1

BUNDESDRUCKEREI BERLIN

Procédé de préparation d'amino-4 chloroquinoléines

La présente invention concerne un procédé de préparation d'une amino-4 chloro-7 quinoléine de formule générale:

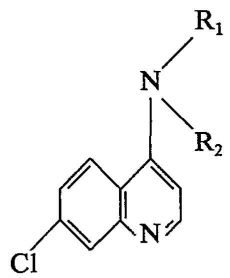

(I)

dans laquelle $R_1$ représente un atome d'hydrogène ou un radical alcoyle contenant 1 à 5 atomes de carbone et $R_2$ représente un radical alcoyle contenant 1 à 5 atomes de carbone éventuellement substitué par un radical dialcoylamino dont chaque partie alcoyle contient 1 à 4 atomes de carbone, tel que le radical diéthylamino-4 méthyl-1 butyle, ou un radical phényle, un radical phényle substitué par un ou plusieurs radicaux choisis parmi les radicaux carboxy, hydroxy ou dialcoylamino alcoyle dont chaque partie alcoyle contient 1 à 4 atomes de carbone, tels que les radicaux carboxy-2 phényle ou diéthylaminométhyl-3 hydroxy-4 phényle, à partir de la chloro-7 tétrahydro-1,2,3,4 quinolinone-4 correspondante de formule générale:

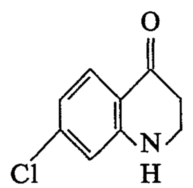

(II)

Les produits de formule générale (I) dans laquelle $R_1$ représente un atome d'hydrogène et $R_2$ représente un radical diéthylamino-4 méthyl-1 butyle ou un radical diéthylaminométhyl-3 hydroxy-4 phényle sont des substances douées de propriétés antimalariques remarquables et sont connues respectivement sous le nom de chloroquine et d'amodiaquine; le produit de formule générale (I), dans laquelle $R_1$ représente un atome d'hydrogène et $R_2$ représente un radical carboxy-2 phényle est un intermédiaire pour la préparation de l'ester dihydroxypropylique de l'acide N-(chloro-7 quinolyl-4) anthranilique connu sous le nom de glafénine qui est un analgésique et un anti-inflammatoire puissant.

Il est connu, en particulier d'après le brevet américain 2 653 940, de préparer la chloroquine par condensation de la diéthylamino-4 méthyl-1 butylamine sur la chloro-7 tétrahydro-1,2,3,4 quinolinone-4 en opérant, de préférence, en présence d'air ou de nitrobenzène comme agent d'oxydation ou de palladium sur charbon comme catalyseur de deshydrogénation.

Cependant cette condensation, conduisant intermédiairement à une base de Schiff, associée à une aromatisation s'accompagne d'une déhalogénation. De cette manière est obtenu un mélange de chloroquine et de (diéthylamino-4 méthyl-1 butylamino)-4 quinoléine.

Il a maintenant été trouvé, et c'est ce qui fait l'objet de la présente invention, que la produit de formule générale (I) peut être obtenu, avec de bons rendements et pratiquement exempt de produit déhalogéné, par condensation d'une amine de formule générale:

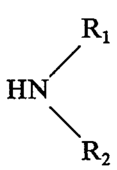

(III)

dans laquelle $R_1$ et $R_2$ sont définis comme précédemment, sur la chloro-7 tétrahydro-1,2,3,4 quinolinone-4 de formule générale (II), associée à une aromatisation, en opérant en présence d'un catalyseur à base de ruthénium sur support et, de préférence, en absence d'oxygène.

La réaction peut être mise en oeuvre en utilisant un catalyseur constitué de ruthénium sur charbon ou sur alumine, et en opérant dans un excès d'amine de formule générale (III) qui peut être utilisée comme solvant, à une température comprise entre 100 et 200° C.

Il est possible aussi d'opérer dans un solvant organique tel que le chlorobenzène ou l'anisole en utilisant un léger excès de l'un ou l'autre des réactifs à la température d'ébullition du solvant ou à une température plus élevée en opérant sous pression et éventuellement en séparant l'eau formée au

cours de la réaction par distillation azéotropique.

Le catalyseur contient de préférence environ 5% en poids de métal et il est utilisé en quantité telle que le métal représente de 0,01 à 0,0001 atome-gramme par mole de réactif mis en oeuvre c'est-à-dire par mole de quinolinone-4 de formule générale (II) ou par mole d'amine de formule générale (III) selon que l'on utilise un excès de quinolinone de formule générale (II) ou d'amine de formule générale (III).

Il peut être avantageux d'opérer en présence d'ions halogénures et plus particulièrement d'ions iodures.

Généralement, on utilise un sel halogéné de l'amine de formule générale (III).

La mise en oeuvre du procédé selon l'invention permet d'obtenir le produit de formule générale (I) ne contenant qu'une faible proportion de produit déhalogéné.

Le taux de produit déhalogéné obtenu par rapport à la tétrahydro-1,2,3,4 quinolinone-4 de formule générale (II) transformée est généralement inférieure à 8% et, selon la qualité de l'amine de formule générale (III) mise en oeuvre ce taux peut être inférieur à 1%.

Le produit de formule générale (I) peut être séparé du mélange réctionnel et purifié par application de méthodes habituelles telles que la cristallisation ou la chromatographie.

La chloro-7 tétrahydro-1,2,3,4 quinolinone-4 de formule générale (II) utilisée commeproduit de départ peut avantageusement être préparée par cyclisation d'un acide chloroanilino-3 propionique au moyen d'un mélange d'acide fluorhydrique et de trifluorure de bore.

L'acide chloroanilino-3 propionique peut être obtenu par action d'un excès de chloroaniline sur l'acide acrylique. La réaction est effectuée dans l'eau à une température comprise entre 70 et 100° C. La durée de la réaction est généralement comprise entre 1 et 4 heures.

Les exemples suivants, donnés à titre non limitatif, montrent comment l'invention peut être mise en pratique.

## Exemple 1

Dans un ballon de 50 $cm^3$ muni d'un agitateur magnétique, d'une colonne à distiller et d'un condenseur, on charge:

| | |
|---|---|
| — catalyseur: composé de 48% en poids de ruthénium sur charbon et de 54% d'eau [teneur en ruthénium dans le catalyseur sec: 5% (p/p)] | 97,3 mg |
| — chloro7 tétrahydro-1,2,3,4 quinolinone-4 | 4,549 g |
| — diéthylamino-1 amino-4 pentane | 3,2655 g |
| — diiodhydrate de diéthylamino-1 amino-4 pentane | 47,2 mg |
| — anisole | 25 $cm^3$ |

La sortie du condenseur est reliée à un gazomètre permettant de mesurer le volume de gaz dégagé au cours de la réaction. On chauffe le mélange réactionnel jusqu'au reflux en 16 minutes. La température du mélange reste alors constante et égale à 157°C pendant toute la durée de l'opération. Les vapeurs sont condensées en tête de colonne et le distillat hétérogène est décanté; le solvant est recyclé en continu.

Après 5 heures de chauffage le volume de gaz dégagé est de 435 $cm^3$.

On rèfroidit, sépare le catalyseur par filtration, et lave la phase organique avec de la soude aqueuse 1 N pour libérer les amines présentes sous forme de sel.

La solution organique ainsi obtenue est examinée par chromatographie en phase gazeuse. On obtient les résultats suivants:

- chloro-7 tétrahydro-1,2,3,4 quinolinone-4: 7,037 mM pour une quantité chargée au départ de 25,063 mM soit un taux de transformation de 71,9%
- diéthylamino-1 amino-4 pentane: 1,518 mM pour une quantité totale au départ de 19,882 + 0,114 = 19,996 mM soit un taux de transformation de 92,4%
- chloroquine: 17,8 mM représentant un rendement (dosé) de 99% par rapport à la chloro-7 tétrahydro-1,2,3,4 quinolinone-4 transformée et de 96,3% par rapport au diéthylamino-1 amino-4 pentane transformé
- (diéthylamino-4 méthyl-1 butylamino)-4 quinoléine: 0,08 mM ce qui correspond à un rendement de 0,45% par rapport à la chloro-7 tétrahydro-1,2,3,4 quinolinone-4 transformée.

La chloro-7 tétrahydro-1,2,3,4 quinolinone-4 de départ peut être préparée de la manière suivante:

Dans un réacteur en acier inoxydable contenant 50 g d'acide fluorhydrique liquide refroidi à 5° C on ajoute 10 g d'acide m.chloroanilino-3 propionique (titrant 94,5%). La solution est saturée de trifluorure de bore gazeux. A cet effet, le contenu du réacteur est maintenu à 20° C puis saturé par le trifluorure de bore gazeux sous une pression de 12 bars pendant 1 heure. Le réacteur est ensuite fermé puis chauffé à 80° C pendant 20 heures.

La pression s'élève d'abord jusqu'à 20 bars puis diminue progressivement pour se stabiliser vers 16 bars. Le réacteur est ensuite refroidi à 10° C puis ouvert de manière à laisser échapper le trifluorure de bore. Le liquide rougeâtre obtenu est versé dans un mélange d'eau et de glace. Après extraction 3 fois par 100 $cm^3$ de chloroforme, la couche organique est lavée plusieurs fois par 100 $cm^3$ d'eau jusqu'à pH compris entre 3 et 4 puis séchée sur sulfate de sodium. Après filtration et concentration à sec sous pression réduite (10 mm de mercure; 1,33 kPa), on obtient 9 g de chloro-7 tétrahydro-1,2,3,4 quinolinone-4 cristallisée dont le titre déterminé par chromatographie en phase gazeuse est 94,5%.

Le taux de transformation est de 100% et le rendement par rapport à l'acide m.chloroanilino-3 propionique est de 99%.

Par examen en chromatographie en phase gazeuse, la teneur en isomère chloro-5 est voisine de 0,7%.

L'acide m.chloroanilino-3 propionique de départ peut être préparé de la manière suivante:

A un mélange de 510,3 g de m.chloroaniline dans 150 $cm^3$ d'eau, maintenu sous atmosphère d'argon et agité à 80° C, on ajoute en 10 minutes une solution de 72,05 g d'acide acrylique dans 100 $cm^3$ d'eau. Le mélange réactionnel constitué de 2 phases est maintenu pendant 3 heures à 80° C sous agitation puis refroidi à 20° C. Après décantation, la phase aqueuse (couche supérieure) est éliminée. A la phase organique on ajoute 423 $cm^3$ d'une solution aqueuse de soude 2,6 N, en agitant et en maintenant la température à 20° C. Aprés décantation la phase organique constituée de 303 g de m.chloroaniline est séparée. La phase aqueuse (850 $cm^3$) est extraite 6 fois successivement par 450 $cm^3$ d'éther.

La phase aqueuse, dont on élimine l'éther par évaporation sous pression réduite (20 mm de mercure, 2,7 kPa), est acidifiée par addition de 105 g d'acide sulfurique à 50% (en poids). Le pH final est 3,5 (point isoélectrique). La température passe de 22 à 33° C puis on chauffe à 40° C. Après décantation, on sépare:

— une phase organique inférieure (208,8 g) constituée d'acide m.chloroanilino-3 propionique fondu saturé d'eau (8,6% d'eau)
— une phase aqueuse supérieure (601 g) contenant 2,28 g d'acide m.chloroanilinopropionique et 156 g de sulfate de sodium.

La phase organique est chauffée pendant 1 heure à 80° C sous pression réduite (20 mm de mercure; 2,7 kPa). On obtient 195,4 g d'un produit contenant 94% d'acide m. chloroanilino-3 propionique et 2,3% d'eau.

Le diéthylamino-1 amino-4 pentane dont le titre est supérieur à 95%, utilisé comme produit de départ, est obtenu après distillation sous une pression de 30 mm de mercure (4 kPa) du produit de réaction de la diéthylamino-5 pentanone-2 avec l'ammoniac et l'hydrogène. La diéthylamino-5 pentanone-2 peut être obtenue par action du diéthylamino-1 chloro-2 éthane sur l'acétylacétate d'éthyle sodé.

## Exemple 2

On opère comme dans l'exemple 1 mais en utilisant un catalyseur préréduit:

On charge le catalyseur (ruthénium à 5%/charbon à 57% d'eau et 43% de catalyseur sec): 98,9 mg soit 0,021 m.At.g de ruthénium.

On chaffe à 100° C puis fait passer un courant d'hydrogène pendant 1 heure. On refroidit puis charge dans le ballon:

| | |
|---|---|
| — chloro-7 tétrahydro-1,2,3,4 quinolinone-4 | 4,5617 g (25,133 mM) |
| — diéthylamino-1 amino-4 pentane (obtenu dans les conditions décrites dans l'exemple 1) | 3,307 g (20,135 mM) |
| — diiodhydrate de diéthylamino-1 amino-4 pentane | 48,7 mg (0,117 mM) |
| — anisole | 25 $cm^3$ |

On procède comme dans l'exemple 1. Après 5 heures de chauffage au reflux le volume de gaz dégagé est de 447 $cm^3$.

Après séparation du catalyseur par filtration et lavage de la phase organique par une solution de soude N, on dose par chromatographie gazeuse:

— chloro-7 tétrahydro-1,2,3,4 quinolinone-4: 6,875 mM ce qui correspondant à un taux de transformation de 72,6%
— diéthylamino-1 amino-4 pentane: 1,769 mM soit un taux de transformation de 91,3%
— chloroquine: 17,904 mM soit un rendement de 98,05% par rapport à la chloro-7 tétrahydro-1,2,3,4 quinolinone-4 transformée et un rendement de 96,86% par rapport au diéthylamino-1 amino-4 pentane transformé
— (diéthylamino-4 méthyl-1 butylamino)-4 quinoléine: 0,08 M ce qui réprésente un rendement de

0,43% par rapport à la chloro-7 tétrahydro-1,2,3,4 quinolinone-4 transformée.

Exemple 3

On opère comme dans l'exemple 1 mais en utilisant les produits suivants:

| | |
|---|---|
| — catalyseur (ruthénium sur charbon à 5% (p/p) à 50% d'eau et 50% de catalyseur sec) | 154,6 mg (0,0382 m.At.g) |
| — chloro-7 tétrahydro-1,2,3,4 quinclinone-4 | 961,5 mg (5,297 mM) |
| — diéthylamino-1 amino-4 pentane (obtenu dans les conditions décrites dans l'exemple 1) | 642,2 mg (3,910 mM) |
| — dichlorhydrate de diéthylamino1 amino-4 pentane | 51,7 mg (0,224 mM) |
| — chlorobenzène | 12 $cm^3$ |

On chauffe au reflux pendant 4 heures 30 minutes la température du mélange réactionnel étant de 136° C.

Après filtration du catalyseur et lavage à la soude dans les conditions décrites dans l'exemple 1 on dose par chromatographie en phase gazeuse:

- chloro-7 tétrahydro-1,2,3,4 quinolinone-4: 1,216 mM soit un taux de transformation de 77%
- diéthylamino-1 amino-4 pentane: 0,115 mM soit un taux de transformation de 97,2%
- chloroquine: 3,665 mM soit un rendement par rapport à la chloro-7 tétrahydro-1,2,3,4 quinolinone-4 transformée de 90% et un rendement par rapport au diéthylamino-1 amino-4 pentane transformé de 91,2%
- (diéthylamino-4 méthyl-1 butylamino)-4 quinoléine: rendement estimé 3,5% par rapport à la chloro-7 tétrahydro-1,2,3,4 quinolinone-4 transformée.

Exemple 4

On opère comme dans l'exemple 1 mais en utilisant les produits suivants:

| | |
|---|---|
| — catalyseur (ruthénium sur charbon à 5% (p/p) 50% d'eau et 50% de catalyseur sec) | 757,8 mg (0,187 m.At.g) |
| — chloro-7 tétrahydro-1,2,3,4 quinolinone-4 | 4,8199 g (26,556 mM) |
| — diéthylamino-1 amino-4 pentane (obtenu dans les conditions décrites dans l'exemple 1) | 3,2465 g (19,767 mM) |
| — dichlorhydrate de diéthylamino-1 amino-4 pentane | 259,1 mg (1,121 mM) |
| — diiodhydrate de diéthylamino-1 amino-4 pentane | 41 mg (0,099 mM) |
| — chlorobenzène | 25 $cm^3$ |

On chauffe pendant 4 heures 50 minutes au reflux; le volume de gaz dégagé est de 470 $cm^3$.

Par analyse chromatographique on dose:

- chloro-7 tétrahydro-1,2,3,4 quinolinone-4: 6,418 mM soit un taux de transformation de 75,8%
- diéthylamino-1 amino-4 pentane: 1,391 mM soit un taux de transformation de 93,4%
- chloroquine: 19,116 mM soit un rendement par rapport à la chloro-7 tétrahydro-1,2,3,4 quinolinone-4 transformée de 94,9% et un rendement de 97,5% par rapport au diéthylamino-1 amino-4 pentane transformée
- (diéthylamino-4 méthyl-1 butylamino)-4 quinoléine (éstimée) correspond à un rendement de 1% par rapport à la chloro-7 tétrahydro-1,2,3,4 quinolinone-4 transformée.

Exemple 5

On opère comme dans l'exemple 1, mais à partir des produits suivants:

| | |
|---|---|
| — catalyseur: ruthénium sur charbon à 2,15% p/p de ruthénium métal | 98,5 mg (0,021 m.At.g) |
| — chloro-7 tétrahydro-1,2,3, 4 quinolinone-4 | 3,6007 g (19,84 mM) |
| — diéthylamino-1 amino-4 pentane, obtenu dans les conditions décrites dans l'exemple 1, titrant 96,2% | 4,118 g (25,03 mM) |
| — diiodhydrate de diéthylamino-1 méthyl-4 pentane | 28,2 mg (0,064 mM) |

— anisole 15 $cm^3$

On opère comme dans l'exemple 1. Après 5 heures 30 de chauffage à reflux, le mélange réactionnel est refroidi. Le catalyseur est séparé par filtration. La phase organique est lavée par une solution aqueuse de soude N. On dose alors dans la phase organique, par chromatographie en phase gazeuse

| | |
|---|---|
| — chloro-7 tétrahydro-1,2,3,4 quinolinone-4 (ce qui correspond à un taux de transformation de 90,2%) | 1,95 mM |
| — diéthylamino-1 amino-4 pentane (ce qui correspond à un taux de transformation de 78,33%) | 5,437 mM |
| — chloroquine (soit un rendement de 99,6% par rapport à la chloro-7 quinolinone-4 transformée et un rendement de 90,7% par rapport au diéthylamino-1 amino-4 pentane transformé) | 17,827 mM |
| — (diéthylamino-4 méthyl-1 butylamino)-4 quinoléine (soit un rendement de 0,36% par rapport à la chloro-7 quinolinone-4 transformée) | 0,064 mM |

Exemple 6

On opère comme dans l'exemple 1, mais à partir des produits suivants:

| | |
|---|---|
| — catalyseur: ruthénium sur alumine à 5% (p/p) | 42,9 mg (0,021 m.At.g) |
| — chloro-7 tétrahydro-1,2,3,4 quinolinone-4 | 4,533 g (24,975 mM) |
| — diéthylamino-1 amino-4 pentane, titrant 96,2% | 3,471 g (21,133 mM) |
| — diiodhydrate de diéthylamino-1 amino-4 pentane | 25,9 mg (0,059 mM) |
| — anisole | 25 $cm^3$ |

On opère comme dans l'exemple 1. Après 7 heures de chauffage au reflux, le mélange réactionnel est refroidi.

Après séparation du catalyseur par filtration et lavage de la phase organique par une solution aqueuse de soude N, on dose, par chromatographie en phase gazeuse:

| | |
|---|---|
| — chloro-7 tétrahydro-1,2,3,4 quinolinone-4 (ce qui correspond à un taux de transformation de 74,8%) | 6,301 mM |
| — diéthylamino-1 amino-4 pentane (ce qui correspond à un taux de transformation de 92,7%) | 1,551 mM |
| — chloroquine (soit un rendement de 96,7% par rapport à la chloro-7 quinolinone-4 transformée ou de 91,9% par rapport au diéthylamino-1 amino-4 pentane transformé) | 18,057 mM |
| — (diéthylamino-4 méthyl-1 butylamino)-4 quinoléine (soit un rendement de 1,37% par rapport à la chloro-7 quinolinone-4 transformée) | 0,257 mM |

Exemple 7

On opère comme dans l'exemple 1, mais à partir des produits suivants:

| | |
|---|---|
| — catalyseur: ruthénium sur charbon à 2,15% (p/p) de ruthénium métal | 98,7 mg (0,021 m.At.g) |
| — chloro-7 tétrahydro-1,2,3,4 quinolinone-4 | 4,5734 g (25,197 mM) |
| — diéthylamino-1 amino-4 pentane | 3,4651 g (21,097 mM) |
| — diiodhydrate de diéthylamino-1 amino-4 pentane | 26,1 mg (0, 057 mM) |
| — anisole | 21 $cm^3$ |

On opère comme dans l'exemple 1. Après 8 heures de chauffage au reflux, le volume de gaz dégagé est de 487 $cm^3$, mesuré à une température voisine de 20°C. Après refroidissement du mélange réactionnel, le catalyseur est séparé par filtration. On lave la phase organique par une solution aqueuse de soude N puis on dose, par chromatographie en phase gazeuse:

| | |
|---|---|
| — chloro-7 tétrahydro-1,2,3,4 quinolinone-4 (soit un taux de transformation de 83,9%) | 4,047 mM |

| | |
|---|---|
| — diéthylamino-1 amino-4 pentane (soit un taux de transformation de 97,7%) | 0,484 mM |
| — chloroquine (soit un rendement de 96,6% par rapport à la chloro-7 quinolinone-4 transformée ou de 98,9% par rapport au diéthylamino-1 amino-4 pentane transformé) | 20,439 mM |
| — (diéthylamino-4 méthyl-1 butylamino)-4 quinoléine (soit un rendement de 0,28% par rapport à la chloro-7 quinolinone-4 transformée) | 0,06 mM |

Exemple 8

On opère comme dans l'exemple 1, mais à partir des produits suivants:

| | |
|---|---|
| — catalyseur: ruthénium sur charbon à 5% (p/p) | 46,7 mg (0,023 m.At.g) |
| — chloro-7 tétrahydro-1,2,3,4 quinolinone-4 | 4,4864 g (24,718 mM) |
| — aniline | 1,9155 g (20, 597 mM) |
| — chlorhydrate d'aniline | 29,9 mg (0,23 mM) |
| — anisole | 25 $cm^3$ |

On opère comme dans l'exemple 1. Après 5 heures de chauffage au reflux, on prélève en 30 minutes, 15 $cm^3$ de distillat, puis on poursuit, le chauffage au reflux pendant encore 3 heures. Pendant la seconde partie de la réaction, la température du mélange réactionnel au reflux est de 170° C. Le volume de gaz dégagé est de 455 $cm^3$, mesuré à une température voisine de 20° C. Un précipité apparaît par refroidissement. Ce précipité est séparé par filtration puis repris par de l'éthanol à chaud. Le catalyseur est séparé par filtration. Le filtrat est concentré à sec. On obtient ainsi 3,55 g d'un solide A.

La phase organique est lavée par une solution aqueuse de soude N puis par une solution d'acide chlorhydrique N. Dans la phase organique ainsi lavée, on dose, par chromatographie en phase gazeuse:

| | |
|---|---|
| — chloro-7 tétrahydro1,2,3,4 quinolinone-4 (soit un taux de transformation de 82,3%) | 4,374 mM |

La solution chlorhydrique aqueuse est alcalinisée par une solution aqueuse de soude N puis est extraite à l'éther. Dans la phase éthérée, on dose, par chromatographie en phase gazeuse:

| | |
|---|---|
| — aniline (soit un taux de transformation de 97,1%) | 0,604 mM |

La phase éthérée est concentrée à sec. Le résidu obtenu et le solide A sont réunis et dissous à chaud dans de l'éthanol. Par refroidissement, on obtient un précipité cristallisé qui est séparé par filtration. On obtient ainsi 2,475 g d'anilino-4 chloro-7 quinoléine fondant à 209° C.

Après concentration du filtrat, on obtient une seconde fraction de 1,325 g d'anilino-4 chloro-7 quinoléine fondant à 207° C.

Par chromatographie en couche mince et par chromatographie en phase gazeuse, le produit isolé est pratiquement pur.

Le rendement en anilino-4 chloro-7 quinoléine isolée est de 73,4% par rapport à la quinolinone-4 transformée et de 73,8% par rapport à l'aniline transformée.

Exemple 9

On opère comme dans l'exemple 1 mais à partir des produits suivants:

| | |
|---|---|
| — catalyseur: ruthénium sur charbon à 5% (p/p) | 45,3 mg (0,022 m.At. g) |
| — chloro-7 tétrahydro-1,2,3,4 quinolinone-4 | 4,5212 g (24,91 mM) |
| — acide anthranilique | 2,9668 g (21,655 mM) |
| — anisole | 15 $cm^3$ |

On chauffe au reflux sous agitation pendant 5 heures.

Pendant la réaction, un précipité apparaît dans le mélange réactionnel. Après refroidissement, le précipité est séparé par filtration puis il est repris par de l'acide acétique à chaud. Le catalyseur est alors séparé par filtration. Après refroidissement, on obtient un précipité cristallisé jaune clair qui est séparé par filtration. Après recristallisation dans le méthanol, on obtient 3,829 g de [(carboxy-2 phényl) amino]-4 chloro-7 quinoléine titrant 95,03%.

Dans le filtrat obtenu à l'issui de la première filtration, on dose, par chromatographie en phase gazeuse:

— chloro-7 tétrahydro-1,2,3,4 quinolinone-4 (ce qui correspond à un taux de transformation de 86,3%) 3,406 mM

La [(carboxy-2 phényl) amino]-4 chloro-7 quinoléine est obtenue avec un rendement de 56,7% par rapport à la quinolinone-4 transformée.

## Exemple 10

Dans l'appareil décrit dans l'exemple 1, on introduit:

| | |
|---|---|
| — catalyseur: ruthénium sur charbon à 5% (p/p) | 49,3 mg (p,024 m.At.g) |
| — chloro-7 tétrahydro-1,2,3,4 quinolinone-4 | 4,5398 g (25,012 mM) |
| — N-méthylaniline | 2,3176 g (21,694 mM) |
| — chlorhydrate de N-méthylaniline | 28,8 mg (0, 2 mM) |
| — anisole | 15 $cm^3$ |

On chauffe au reflux pendant 5 heures puis on prélève 5 $cm^3$ de distillat. On poursuit le chauffage pendant encore 2 heures 30. Le volume de gaz dégagé est de 477 $cm^3$.

Après refroidissement, il se forme un précipité qui est séparé par filtration puis est repris dans une solution aqueuse de soude N. Le catalyseur est séparé par filtration. Le filtrat organique de la première filtration est lavé par la soude aqueuse N ayant servi à la reprise du précipité. Après acidification de la phase aqueuse à pH 6 et extraction au butanol, on obtient 1,43 g de chloro-7 hydroxy-4 quinoléine.

La phase organique est ensuite lavée par de l'acide chlorhydrique N. Dans la phase organique, on dose, par chromatographie en phase gazeuse:

— chloro-7 tétrahydro-1,2,3,4 quinolinone-4 (ce qui correspond à un taux de transformation de 79,3%) 5,175 mM

La solution aqueuse acide est alcalinisée par addition de soude N puis est extraite à l'éther. Dans cet extrait éthéré, on dose, par chromatographie en phase vapeur:

— N-méthylaniline (ce qui correspond à un taux de transformation de 58,8%) 9,013 mM

Après avoir évaporé l'éther, le résidu obtenu est recristallisé dans l'hexane. On obtient ainsi 2,117 g de (N-méthyl N-phénylamino)-4 chloro-7 quinoléine.

Le rendement est de 40% par rapport à la quinolinone-4 transformée et de 61% par rapport à la N-méthylaniline transformée.

## Exemple 11

Dans l'appareil décrit à l'exemple 1, on introduit:

| | |
|---|---|
| — catalyseur: ruthénium sur charbon à 5% (p/p) | 48,9 mg (0,024 m.At.g) |
| — chloro7 tétrahydro-1,2,3,4 quinolinone-4 | 4,572 g (25,19 mM) |
| — di (n.butyl) amine | 17 $cm^3$ |
| — chlorhydrate de di (n.butyl) amine | 344,4 mg (2,08 mM) |
| — chlorobenzène | 2 $cm^3$ |

On chauffe à reflux pendant 11 heures. Après refroidissement, le catalyseur est séparé par filtration. Le filtrat additionné de chlorobenzène est lavé par une solution aqueuse de soude N. La phase organique est lavée par une solution d'acide chlorhydrique N. Dans la phase organique, on dose, par chromatographie en phase gazeuse:

— chloro-7 tétrahydro1,2,3,4 quinolinone-4 (ce qui correspond à un taux de transformation de 87,4%) 3,17 mM

La solution chlorhydrique est alcalinisée par addition d'une solution aqueuse de soude N puis est extraite à l'éther. Après concentration de la phase éthérée sous pression réduite, on obtient 4,73 g d'une huile constituée essentiellement de N,N-dibutylamino-4 chloro-7 quinoléine.

Exemple 12

Dans l'appareil décrit dans l'exemple 1, on charge:

| | |
|---|---|
| — catalyseur: ruthénium sur charbon à 5% (p/p) | 47,7 mg (0,023 m.At.g) |
| — chloro-7 tétrahydro-1,2,3,4 quinolinone-4 | 4,454 g (24,54 mM) |
| — p.aminophénol | 2,2247 g (20,41 mM) |
| — iodhydrate de p.aminophénol | 51,5 mg (0,022 mM) |
| — anisole | 25 $cm^3$ |

On chauffe à reflux pendant 17 heures. Après refroidissement, il se forme un précipité qui est séparé par filtration puis qui est dissous dans de l'éthanol à chaud. Le catalyseur est alors séparé par filtration.

Par addition d'eau à chaud dans le filtrat alcoolique, il se forme un précipité jaune pâle qui est séparé par filtration. On obtient ainsi 3,006 g d'(hydroxy-4 phényl) amino-4 chloro-7 quinoléine fondant à 257° C.

Par concentration partielle du filtrat, on obtient 0,605 g d'(hydroxy-4 phényl) amino-4 chloro-7 quinoléine fondant à 255° C.

La solution contenant l'anisole, issue de la première filtration, est lavée par une solution aqueuse d'acide sulfurique 2 N. Dans la phase organique, on dose, par chromatographie en phase gazeuse:

| | |
|---|---|
| — chloro-7 tétrahydro-1,2,3,4 quinolinone-4 (ce qui correspond à un taux de transformation de 79,45%) | 5,043 mM |

La phase aqueuse est amenée à pH 8. Après concentration et extraction, on dose 3,685 mM de p.aminophénol (ce qui correspond à un taux de transformation de 81,9%) et on isole, après cristallisation dans un milieu hydroalcoolique, 0,310 g d'(hydroxy-4 phényl) amino-4 chloro-7 quinoléine fondant à 255° C.

Le rendement en (hydroxy-4 phényl) amino-4 chloro-7 quinoléine est de 74,35% par rapport à la chloro-7 tétrahydro-1,2,3,4 quinolinone-4 transformée et de 86,6% par rapport au p.aminophénol transformé.

L'(hydroxy-4 phényl) amino-4 chloro-7 quinoléine peut être transformée en amodiaquine selon la méthode décrite par J. H. Burckhacter et Coll., J. Amer. Chem. Soc., 72, 1024 (1950).

**Revendications**

1. Procédé de préparation d'une amino-4 chloro-7 quinoléine de formule générale:

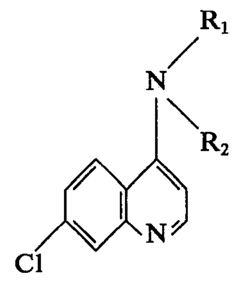

dans laquelle $R_1$ représente un atome d'hydrogène ou un radical alcoyle contenant 1 à 5 atomes de carbone et $R_2$ représente un radical alcoyle contenant 1 à 5 atomes de carbone éventuellement substitué par un radical dialcoylamino dont chaque partie alcoyle contient 1 à 4 atomes de carbone, ou un radical phényle, un radical phényle substitué par un ou plusieurs radicaux choisis parmi les radicaux carboxy, hydroxy ou dialcoylaminoalcoyle dont chaque partie alcoyle contient 1 à 4 atomes de carbone, par condensation associée à une aromatisation d'une amine de formule générale:

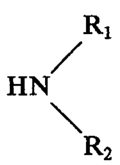

dans laquelle $R_1$ et $R_2$ sont définis comme précédemment, sur une chloro-7 tétrahydro-1,2,3,4 quinolinone-4 de formule générale:

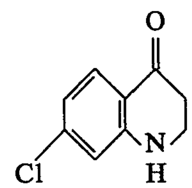

caractérisé en ce que l'on opère en présence d'un catalyseur à base de ruthénium sur support.

2. Procédé selon la revendication 1 caractérisé en ce que l'on opère en absence d'oxygène.

3. Procédé selon la revendication 1 ou 2 caractérisé en ce que l'on effectue la condensation et l'aromatisation en présence de ruthénium sur charbon ou sur alumine éventuellement dans un solvant organique à une température comprise entre 100 et 200° C.

4. Procédé selon la revendication 3, caractérisé en ce que le solvant organique est choisi parmi le chlorobenzène et l'anisole.

5. Procédé selon la revendication 3, caractérisé en ce que l'on opère sous pression.

6. Procédé selon la revendication 3, caractérisé en ce que l'on élimine l'eau formée au cours de la réaction par distillation azéotropique.

7. Procédé selon la revendication 1, 2 ou 3 caractérisé en ce que l'on opère en présence d'ions halogénures.

8. Procédé selon la revendication 7, caractérisé en ce que l'on opère en présence d'un sel halogéné d'une amine de formule générale:

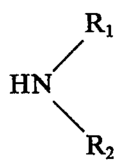

dans laquelle $R_1$ et $R_2$ sont définis comme dans la revendication 1.

9. Procédé selon la revendication 1, caractérisé en ce que l'on fait réagir la chloro-7 tétrahydro-1,2,3,4 quinolinone-4 avec la diéthylamino-4 méthyl-1 butylamine et isole la chloroquine ainsi obtenue.

**Patentansprüche**

1. Verfahren zur Herstellung eines 4-Amino-7-chlorchinolins der allgemeinen Formel:

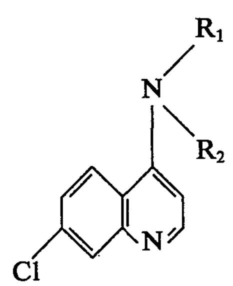

worin $R_1$ ein Wasserstoffatom oder ein Alkylrest mit 1—5 Kohlenstoffatomen ist und $R_2$ ein Alkylrest mit 1—5 Kohlenstoffatomen ist, der gegebenenfalls durch einen Dialkylaminorest substituiert ist, wovon jeder Alkylteil 1—4 Kohlenstoffatome enthält, oder ein gegebenenfalls durch einen oder mehrere Reste substituierter Phenylrest ist, welche Reste ausgewählt sind aus den Carboxy-, Hydroxy- oder Dialkylaminoalkylresten, wovon jeder Alkylteil 1—4 Kohlenstoffatome enthält, durch Kondensation und gleichzeitige Aromatisierung eines Amins der allgemeinen Formel:

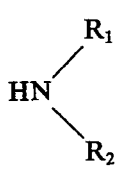

worin $R_1$ und $R_2$ wie vorstehend definiert sind, mit einem 7-Chlor-1,2,3,4-tetrahydrochinolin-4-on der

allgemeinen Formel:

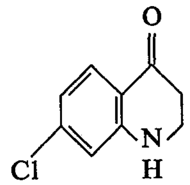

dadurch gekennzeichnet, daß man in Gegenwart eines Katalysators auf Basis von Ruthenium auf einem Träger arbeitet.

2. Verfahren nach Anspruch 1, dadurch gekennzeichnet, daß man in Abwesenheit von Sauerstoff arbeitet.

3. Verfahren nach Anspruch 1 oder 2, dadurch gekennzeichnet, daß man die Kondensation und die Aromatisierung in Gegenwart von Ruthenium auf Kohle oder Aluminiumoxid gegebenenfalls in einem organischen Lösungsmittel bei einer Temperatur zwischen 100 und 200° C durchführt.

4. Verfahren nach Anspruch 3, dadurch gekennzeichnet, daß das organische Lösungsmittel aus Chlorbenzol und Anisol ausgewählt wird.

5. Verfahren nach Anspruch 3, dadurch gekennzeichnet, daß man unter Druck arbeitet.

6. Verfahren nach Anspruch 3, dadurch gekennzeichnet, daß man das während der Umsetzung gebildete Wasser durch azeotrope Destillation entfernt.

7. Verfahren nach Anspruch 1, 2 oder 3, dadurch gekennzeichnet, daß man in Gegenwart von Halogenidionen arbeitet.

8. Verfahren nach Anspruch 7, dadurch gekennzeichnet, daß man in Gegenwart eines halogenierten Salzes eines Amins der allgemeinen Formel:

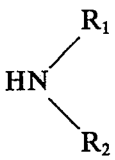

arbeitet, worin $R_1$ und $R_2$ wie in Anspruch 1 definiert sind.

9. Verfahren nach Anspruch 1, dadurch gekennzeichnet, daß man das 7-Chlor-1,2,3,4-tetrahydrochinolin-4-on mit dem 4-Diäthylamino-1-methylbutylamin umsetzt und das so erhaltene Chloroquin isoliert.

**Claims**

1. Process for the preparation of a 4-amino-7-chloroquinoline of the general formula:

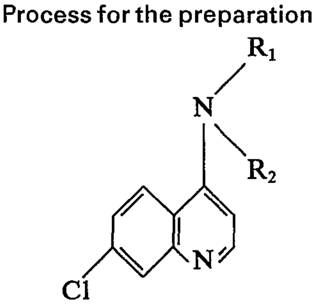

in which $R_1$ represents a hydrogen atom or an alkyl radical containing 1 to 5 carbon atoms and $R_2$ represents an alkyl radical containing 1 to 5 carbon atoms, optionally substituted by a dialkylamino radical of which each alkyl part contains 1 to 4 carbon atoms, or represents a phenyl radical which is unsubstituted or substituted by one or more radicals chosen from amongst carboxy, hydroxy, or dialkylaminoalkyl radicals of which each alkyl part contains 1 to 4 carbon atoms, by condensation accompanied by aromatisation of an amine of the general formula:

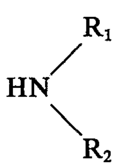

in which $R_1$ and $R_2$ are as defined above, with a 7-chloro-1,2,3,4-tetrahydroquinolin-4-one of the general

formula:

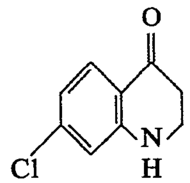

characterized in that it is carried out in the presence of a ruthenium based catalyst on a support.

2. Process according to claim 1, characterized in that it is carried out in the absence of oxygen.

3. Process according to claim 1 or 2, characterized in that the condensation and the aromatisation are carried out in the presence of ruthenium on charcoal or on alumina, optionally in an organic solvent, at a temperature between 100 and 200° C.

4. Process according to claim 3, characterized in that the organic solvent is chosen from amongst chlorobenzene and anisole.

5. Process according to claim 3, characterized in that it is carried out under pressure.

6. Process according to claim 3, characterized in that the water formed during the reaction is removed by azeotropic distillation.

7. Process according to claim 1, 2 or 3, characterized in that it is carried out in the presence of halide ions.

8. Process according to claim 7, characterized in that it is carried out in the presence of a halogen-containing salt of an amine of the general formula:

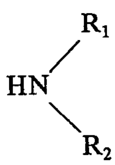

in which $R_1$ and $R_2$ are as defined in claim 1.

9. Process according to claim 1, characterized in that 7-chloro-1,2,3,4-tetrahydroquinolin-4-one is reacted with 4-diethylamino-1-methyl-butylamine and the chloroquine thus obtained is isolated.